Europäisches Patentamt

European Patent Office

Office européen des brevets

(11) Publication number: **0 286 447 B1**

# EUROPEAN PATENT SPECIFICATION

(45) Date of publication of patent specification: 29.09.93   (51) Int. Cl.5: **A61K 39/00**, A61K 37/02

(21) Application number: **88303198.1**

(22) Date of filing: **11.04.88**

(54) Method and agents relating to prophylactic treatment of autoimmune diseases.

(30) Priority: **09.04.87 US 36372**

(43) Date of publication of application:
**12.10.88 Bulletin  88/41**

(45) Publication of the grant of the patent:
**29.09.93 Bulletin  93/39**

(84) Designated Contracting States:
**AT BE CH DE ES FR GB GR IT LI NL SE**

(56) References cited:
**WO-A-86/04093**
**WO-A-86/07464**

**Nature, vol. 324, 18 December 1986, pages 676-679, Shaokee Wu et al "Polymorphism of human Ia antigens generated by reciprocal intergenic exchange between two DR beta loci"**

**Immunogenetics vol. 24, 1986, pages 251-258, New York, S.L. Holbeck et al "Exon-specific oligonucleotide probes localize HLA-DO beta allelic polymorphisms"**

**Nature, vol. 329, 15 October 1987, pages 599-604, J.A. Todd et al "HLA-DObeta gene contributes to susceptibility and resistance**

**to insulin-dependent diabetes mellitus"**

(73) Proprietor: **THE BOARD OF TRUSTEES OF THE LELAND STANFORD JUNIOR UNIVERSITY Encina 105 Stanford University Stanford California 94305(US)**

(72) Inventor: **Todd, John A. Dept.of Medical Microbiology**
**D345 Fairchild Building Stanford University Stanford California 94305(US)**
Inventor: **Bell, John I. The Nuffield Dept. of Clinic Medicine John Radcliffe Hospital**
**Headington Oxford OX3 9DU(GB)**
Inventor: **Acha-Orbea, Hans**
**510 Oak Grove Avenue No. B Menlo Park California 94025(US)**
Inventor: **McDevitt, Hugh O. Dept. of Medical Microbiology**
**D345 Fairchild Building Stanford University Stanford California 94305(US)**

(74) Representative: **Harrison, David Christopher et al**
**MEWBURN ELLIS 2 Cursitor Street London EC4A 1BO (GB)**

**Description**

REFERENCE TO GOVERNMENT GRANT

The United States Government has rights to this invention pursuant to Grant No. AM 33880, and Grant No. CA 39069, both awarded by the National Institutes of Health.

DESCRIPTION

The invention is in the field of immunotherapy. More specifically, it relates to controlling autoimmune diseases associated with a particular allele of certain regions of the major histocompatibility complex (MHC), such as the HLA-D region in humans, by selectively suppressing the immune response(s) controlled by the allele with tolerizing amounts of an MHC-encoded peptide which contains an epitope similar in structure to the epitope of the self-antigen involved in the autoimmune reaction.

The major histocompatibility complex (MHC) is a genetic region whose products are primarily responsible for the rapid rejection of grafts between individuals; the products also function in signalling between lymphocytes and cells expressing antigen. All mammals contain an MHC region.

The major histocompatibility complex (MHC) of humans is a cluster of genes occupying a region located on the sixth chromosome. This complex, denoted HLA (Human Leukocyte Antigen), has been divided into five major gene loci, which according to World Health Organization nomenclature are designated, HLA-A, HLA-B, HLA-C, HLA-D, and HLA-DR. The A, B, and C loci are single gene loci. The D and DR loci are multi-gene loci. The A, B, and C loci encode the classical transplantation antigens, whereas the D and DR loci encode products that control immune responsiveness. The gene products of the HLA loci are divided into three classes (I, II, III) based on structure and function (Nature (1982) 297:629-632). Class I encompasses the products of the HLA-A, HLA-B, and HLA-C loci and the Qa/TL region. The Products of HLA-D and HLA-DR (D-related) genes fall in Class II. These include HLA-DP (formerly SB), HLA-DQ (formerly DC) and HLA-DR. The third class, Class III, includes components of complement. As used herein, the term "HLA" is intended to include the above described loci as well as loci that are closely linked thereto.

In mice, the MHC region is referred to as H-2, and is located on chromosome 17. The H-2 complex is divided into four regions, K, I, S and D. It appears that the K and D regions in the mouse perform analogous functions to the A and B regions in man, that is, they act as cell surface recognition molecules which can be identified by cytotoxic T cells. The Mouse I-region is analogous to the D region of man, and contains genes for Class II proteins, which are involved in cooperation and interaction between cells of the immune system. Products of the mouse I region are termed Ia antigens, which is also the generic term of antigens encoded by the human D region. It is found that the strength of an immune response in mice is partly determined by immune response genes (Ir-genes) which map to the H-21 region.

Both Class I and Class II molecules are heterodimeric cell-surface glycoprotein antigens. Class I antigens are composed of 44 kd transmembrane glycoproteins encoded in the MHC noncovalently associated with the 12 kd protein $\beta_2$-microglobulin (which is encoded on human chromosome 15). The Class I antigens, HLA-A, HLA-B, and HLA-C are highly polymorphic, are found on virtually all nucleated cells, and function as targets for cytotoxic T lymphocytes. The Class I antigens, TL and Qa, are much less polymorphic, are found on some lymphocyte and erythrocyte populations and are less well defined than the other Class I antigens.

The Class II antigens, which are highly polymorphic, are involved in communication between cells that regulate the immune response to foreign and self protein antigens. Class II antigenic determinants are generally found as cell-surface antigens composed of two transmembrane glycoproteins of 27-29 kd (light or $\beta$-chain) and 33-35 kd (heavy or $\alpha$-chain). Class II antigens are found primarily on B lymphocytes and on a significant fraction of activated T lymphocytes. Large amounts are also found on so-called antigen-presenting cells, such as dendritic cells and cells of the macrophage/myeloid series.

The HLA system is extremely polymorphic, more so than any other known human polymorphism. At least 23 alleles have been defined for the HLA-A locus, 47 for the B locus, 8 for C, 19 for D, and 14 for DR. There is no single very common allele; rather there are quite a few alleles with frequencies which are relatively even. A large number of allelic forms of the individual Class II genes have been cloned and sequenced (Cf. R.N. Germain and B. Malissen, Ann. Rev. Immunol. 1986. 4:281-315). Comparison of the nucleotide sequences, and especially of the deduced amino acid sequences, revealed that for HLA-DP$\alpha$, HLA-DQ$\beta$, and DR$\beta$ of man, the allelic forms of each gene differ substantially from one another. The allelic polymorphism is concentrated in the amino terminal ($\beta_1$ or $\alpha_1$) domain in each case, and within the domain,

EP 0 286 447 B1

in three or four hypervariable regions spread across the domain.

The ability of an individual to mount an immune response against an antigen is, in part, determined by the Class I and Class II products. These heterodimeric glycoproteins function as receptors on the surface of lymphoid cells or macrophages, for foreign antigen, so that the HLA molecule-antigen complex can be recognized by the antigen receptor specific for T cells. The result of this recognition event is the triggering of the T cell to produce lymphokines and to proliferate, providing stimulation for antibody producing B cells and effector T cells.

The foreign antigen may be protein. The antigenic protein is degraded to peptides in the antigen presenting cell, and certain of the peptides of the antigen may associate with HLA molecules. The ability of one of the HLA receptors to interact with a particular peptide is dependent on the amino acid sequences of the HLA molecules, and the peptide. The potential of an individual's immune response is therefore determined by the difference in the amino acid sequences of the HLA molecules. The individual variation or polymorphism in these molecules is clustered in the hypervariable regions, which are probably physically involved in the peptide-HLA molecule interaction, and hence are central to the immune response.

Autoimmune diseases are the result of an immune response directed against a self antigen. Susceptibility to many of these diseases are associated with serologically defined alleles of the HLA system. The following Tables presents a list of diseases which are known to be associated with specific HLA antigens (Table 1), and the antigen(s) showing the strongest association with particular diseases (Table 2).

Table 1
Diseases Known to be Associated
with Specific HLA Antigens

Rheumatology
Ankylosing spondylitis

Reiter's disease

Yersinia arthritis
Salmonella arthritis
Shigella arthritis

Psoriatic arthritis
Frozen shoulder
Juvenile rheumatoid
  arthritis
Acute anterior uveitis
Rheumatoid arthritis
Rheumatic heart disease

Neurology
Multiple sclerosis
Optic neurotis
Myasthenia gravis
Paralytic poliomyelitis
Schizophrenia
Manic depressive disorder

Dermatology
Psoriasis vulgaris
Psoriasis unspecified
Pustular psoriasis
Dermatitis herpetiformes
Pemphigus
Bechet's disease
Recurrent herpes labialis
Alopeica areata

Allergology
Dust allergy
Rye grass group I allergy
Avian hypersensitivity
Hayfever
Ragweed allergy
Grass pollinosis

Endocrinology
Juvenile insulin-dependent
  diabetes
Thyrotoxicoses (Graves'
  disease)
Hashimoto's thyroditis
de Quervain's thyroiditis
Congential adrenal
  hyperplasia
Idiopathic Addison's disease

Gastroenterology

Coeliac disease
Ulcerative colitis
Crohn's disease
Pernicious anaemia
Atrophic gastritis
Autoimmune chronic active
  hepatitis
Hepatitis B associated
  chronic active hepatitis
Idiopathic haemochromatosis
Alcoholic cirrhosis
Chronic alcoholic
  pancreatitis
Immunopathology
Systematic lupus
  erythematosus
Sicca syndrome
Goodpasture's syndrome
IgA nephropathy
$C_2$ deficiency

Malignant diseases
Retinoblastoma
Hodgkin's disease
Acute lymphatic leukaemia
Nasopharyngeal carcinoma

Other diseases
Congential heart
  malformation
Polycystic kidney disease
Appendicitis acuta
Asbestosis
Cryptogenic fibrosing
  alveolitis

4

Table 2
Antigen Association Data for
Some of the HLA-Associated Diseases

| Disease | HLA | Pati- ents | Con- trols | Rela- tive risk | Attribu- table risk (%) |
|---|---|---|---|---|---|
| Hodgkin's disease | A1 | 40 | 32.0 | 1.4 | 0.12 |
| Idiopathic haemo- chromatosis | A3 | 76 | 28.2 | 8.2 | 0.67 |
| Behcet's disease | B5 | 41 | 10.1 | 6.3 | 0.34 |
| Congenital adrenal hyperplasia | B47 | 9 | 0.6 | 15.4 | 0.08 |
| Ankylosing spondylitis | B27 | 90 | 9.4 | 87.4 | 0.89 |
| Reiter's disease | B27 | 79 | 9.4 | 37.0 | 0.77 |
| Acute anterior uveitis | B27 | 52 | 9.4 | 10.4 | 0.47 |
| Subacute thyroiditis | B35 | 70 | 14.6 | 13.7 | 0.65 |
| Psoriasis vulgaris | Cw6 | 87 | 33.1 | 13.3 | 0.81 |
| Dermatitis herpetiformis | D/DR3 | 85 | 26.3 | 10.8 | 0.72 |
| Coeliac disease | D/DR3 | 79 | 26.3 | 15.4 | 0.80 |
| Sicca syndrome | D/DR3 | 78 | 26.3 | 9.7 | 0.70 |
| Idiopathic Addison's disease | D/DR3 | 69 | 26.3 | 6.3 | 0.58 |
| Graves' disease | D/DR3 | 56 | 26.3 | 3.7 | 0.42 |
| Insulin-dependent diabetes | D/DR3 | 56 | 28.2 | 3.3 | 0.39 |
| | D/DR4 | 75 | 32.2 | 6.4 | 0.63 |
| | D/DR2 | 10 | 30.5 | 0.2 | – |
| SLE | D/DR3 | 70 | 28.2 | 5.8 | 0.58 |
| Multiple sclerosis | D/DR2 | 59 | 25.8 | 4.1 | 0.45 |
| Optic neuritis | D/DR2 | 46 | 25.8 | 2.4 | 0.27 |
| Rheumatoid arthritis | D/DR4 | 50 | 19.4 | 4.2 | 0.38 |
| IgA nephropathy | D/DR4 | 49 | 19.5 | 4.0 | 0.37 |
| Hydralazine-induced SLE | D/DR4 | 73 | 32.7 | 5.6 | 0.60 |
| Hashimoto's thyroiditis | D/DR5 | 19 | 6.9 | 3.2 | 0.13 |
| Pernicious anaemia | D/DR5 | 25 | 5.8 | 5.4 | 0.20 |
| Pauciarticular onset juvenile rheumatoid arthritis | D/DR5 | 50 | 16.2 | 5.2 | 0.40 |

In autoimmune diseases, the presence of an HLA allele which confers susceptibility to the disease is usually dominant, i.e. the presence of one allele linked to the susceptible type is sufficient to render an individual susceptible. However, certain alleles also have a negative correlation with a disease. Moreover, the presence of an allele with a negative correlation is dominant over one which confers susceptibility. For example, with insulin-dependent diabetes mellitus (IDDM), at least 90% of diabetic individuals are DR3 or DR4. McDonald, M.J. et al (1986), Proc. Natl. Acad. Sci., U.S.A. 83, 7049. However, not all DR3 or DR4 individuals are diabetic. Individuals carrying the DR3 or DR4 allele which confers susceptibility who also carry a DR2 allele have significantly lessened manifestation of the disease. McDonald, M.J. et al (1986), Id.

At the molecular level, certain DNA sequences are thought to be correlated to the presence of autoimmune diseases. In a comparison of restriction enzyme polymorphisms between IDDM diabetics and normal controls, it could be shown that certain restriction enzyme fragments which hybridized to an HLA-DQ$\beta$-chain cDNA probe were present with increased frequency in the diabetics. In addition, other restriction fragments had a negative correlation with respect to diabetes. These latter fragments, however, did not appear to be related to DR2, and the genes from which these fragments derive have not been identified.

EP 0 286 447 B1

Owerbach, D. et al (1983), Nature 303: 815.

One method of treating autoimmune disease is by immunosuppression. General immunosuppression is commonly achieved through treatment with a means and/or agent such as radiation, antimitotics, heterologous antilymphocyte sera, heterologous anti-T cell antibody, adrenal steroids, and cytotoxic chemicals. Such treatment is non-specific in the sense that it suppresses the entire immune system rather than a single immune response. The major side effect of nonspecific or general immunosuppression is immunodeficiency which leaves the treated individual highly susceptible to bacterial, viral, and fungal infections that would otherwise be manageable, but under the circumstances are potentially life-threatening.

A more specific type of immunosuppression may be achieved with anti-DR antibodies. However, this therapy has several real and potential side effects which makes its application problematic at the present time.

In summary, methods by which genetically controlled, MHC associated autoimmune diseases may be controlled via selective immunosuppression are highly desirable. The problems addressed by the present invention relate to such methods and agents/compositions for use in them.

One aspect of the invention relates to methods of prophylactically treating an individual for an autoimmune disease to which the individual is susceptible, the autoimmune disease being one to which susceptibility is associated with a first MHC type allele, and to which nonsusceptibility is associated with a second MHC type allele which is protective against the disease. Protection in this instance may be due to antigenic similarity between the self MHC molecule and the self molecule(s) which is (are) the targets o the autoimmune process. In this method, the individual is treated by administering a tolerizing amount of peptide which is encoded within the protective allele, and this causes the protective effect.

Another aspect of the invention is a method for prophylactically treating a human for IDDM, to which the human is susceptible, wherein the susceptibility is associated with a first HLA type allele, and wherein a second HLA type allele is protective against the disease. In this method, the human is treated by administering a tolerizing amount of a peptide encoded within the protective HLA allele, which causes the protective effect.

Still another aspect of the invention is a polypeptide for prophylactically treating an individual for an autoimmune disease of the above described type, this being a peptide encoded within the protective MHC allele, which causes the protective effect. The peptide may be present in a pharmaceutical composition containing a suitable proportion thereof so as to allow dosage of a tolerizing amount.

Another aspect of the invention is a pharmaceutical composition for prophylactically treating a human for IDDM, to which the human is susceptible, the susceptibility being associated with a first HLA type allele, and wherein a second HLA type allele is protective against IDDM. The pharmaceutical composition is comprised of a tolerizing amount of a peptide encoded within the protective HLA allele, wherein the antigen causes the protective effect. The peptide itself is also covered.

The invention also extends in another aspect to use of the appropriate peptide, encoded within the protective MHC (HLA, as the case may be) allele, in a method of making a composition or agent for prophylactically treating susceptible individuals as described above. The polypeptide containing the relevant protective epitope may be prepared by chemical synthesis, according to the amino acid sequence for the epitope derived from the DNA sequence of the protective allele.

In another aspect, it may be produced by expressing a recombinant vector encoding the polypeptide in a compatible host, and then isolating the polypeptide.

In particular, either method may be used to produce a polypeptide containing a protective epitope against IDDM, whose amino acid sequence is encoded within DR2 DQ1 (Dw2) or DR DQ1 (Dw12) or DR4 DQw 3.1.

Yet another aspect of the invention is a method for determining whether an individual should be prophylactically treated for an autoimmune disease, the autoimmune disease being of the above described type. The method comprises determining whether an individual has the allele conferring susceptibility; and for these potentially susceptible individuals, determining whether a protective allele is absent.

Brief Description of the Drawings

Figure 1 presents the amino acid sequences of the first domains of DQ$\beta$3.1 and DQ$\beta$3.2.

Figure 2A presents the amino acid sequence of the first domain of those alleles linked to IDDM susceptibility, and to protection against susceptibility.

Figure 2B presents the amino acid sequences of residues 36-68 present in the alleles which confer susceptibility and non-susceptibility to IDDM, and which are protective and not protective against IDDM manifestation.

6

The practice of the described embodiments employs, unless otherwise indicated, conventional techniques of molecular biology, microbiology, recombinant DNA, and immunology, which are within the skill of the art. Such techniques are explained fully in the literature. See e.g., Maniatis, Fritsch & Sambrook, MOLECULAR CLONING: A LABORATORY MANUAL (1982); DNA CLONING, Volumes I and II (D.N. Glover ed. 1985); OLIGONUCLEOTIDE SYNTHESIS (M.J. Gait ed. 1984); NUCLEIC ACID HYBRIDIZATION (B.D. Hames & S.J.Higgins eds. 1984); TRANSCRIPTION AND TRANSLATION (B.D. Hames & S.J. Higgins eds 1984); ANIMAL CELL CULTURE (R.K. Freshney ed. 1986); IMMOBILIZED CELLS AND ENZYMES (IRL press, 1986); B. Perbal, A PRACTICAL GUIDE TO MOLECULAR CLONING (1984); the series, METHODS IN ENZYMOLOGY (S. Colowick and N. Kaplan eds., Academic Press, Inc.), and HANDBOOK OF EXPERIMENTAL IMMUNOLOGY, Volumes I-IV (D.M. Weir and C. C. Blackwell eds., 1986, Blackwell Scientific Publications).

In describing the present invention, the following terminology will be used in accordance with the definitions set out below.

"Tolerance" is a mechanism that prevents organisms from mounting immune responses to the antigenic determinants of their own macromolecules. "Tolerizing" an individual is inducing a state of "Tolerance" in that individual to a specific antigen.

The "Major Histocompatibility Complex" (MHC) denotes a region of the genome which encodes proteins involved in immunological recognition. The MHC complex in humans is termed the "HLA" complex.

A "protective peptide" is one which is encoded within the MHC region, and which contains an epitope with a structural resemblance to a self-antigen which is involved in the autoimmune response, such that the presence of the "protective peptide" tolerizes the individual to that self-antigen. A "protective fragment" of the "protective peptide" is that portion of the peptide which contains the epitope with the structural resemblance to the epitope of the above described self-antigen, and which is capable of inducing tolerance to it. A "mutant of a protective peptide" is' expressed from a sequence derived from that encoding the protective antigen; the mutated sequence may arise by deletion of a portion of the sequence, by addition of nucleotides, and by substitution of a nucleotide or nucleotides; however, the mutations which give rise to the mutant are engineered, and are not ones which arise in nature. The "mutant peptide", however, contains an epitope of the "protective peptide" which confers the protective effect. As used herein, a "protective peptide" includes a "protective fragment" of the peptide, moreover, "peptide does not connote length of the amino acid chain, but includes polypeptides, oligopeptides, and peptides.

"Antibody" refers to a member of a family of glycosylated proteins called immunoglobulins, which can specifically combine with an antigen.

"Antigen" refers to a protein or peptide compound which will produce antibody formation without chemical modification.

"Epitope" refers to the actual site of antibody recognition of the antigen. The term is also used interchangeably with "antigenic determinant" or "antigenic determinant site" .

"Carrier" is a material to which the antigen is bound or conjugated, and which presents it as a recognizable immunogen to the immune system.

"Conjugate" refers to an antigen or hapten chemically bonded to a carrier; a conjugate can contain other groups, as well.

A "nucleotide construct" refers to the polymeric form of nucleotides, either ribonucleotides or deoxyribonucleotides. This term refers only to the primary structure of the molecule. Thus, this term includes double- and single-stranded DNA as well as double- and single-stranded RNA.

A "replicon" is any genetic element (e.g., a plasmid, a chromosome, a virus) that behaves as an autonomous unit of polynucleotide replication within a cell; i.e. capable of replication under its own control.

A "vector" is a replicon in which another polynucleotide segment is attached, so as to bring about the replication and/or expression of the attached segment.

A "coding sequence" is a polynucleotide sequence which is transcribed and/or translated into a polypeptide when placed under the control of appropriate regulatory sequences. The boundaries of the coding sequence are determined by a translation start codon at the $5'$-terminus and a translation stop codon at the $3'$-terminus. A coding sequence can include, but is not limited to, prokaryotic sequences, cDNA from eukaryotic mRNA, genomic DNA sequences from eukaryotic DNA, and even synthetic DNA sequences. A polyadenylation signal and transcription termination sequence will usually be located $3'$ to the coding sequence.

A "promoter sequence" is a DNA regulatory region capable of binding RNA polymerase and initiating transcription of a downstream (i.e. in the $3'$ direction) coding sequence. In the present invention, the promoter sequence is bounded at its $3'$-terminus by the translation start codon of a coding sequence and extends upstream (i.e. in the 5' direction) to include the minimum number of bases or elements necessary to initiate transcription at levels detectable above background. Within the promotor sequence will be found a transcription initiation site (conveniently determined by mapping with nuclease S1), as well as protein binding domains (consensus sequences) responsible for the binding of RNA polymerase and regulatory proteins.

A coding sequence is "under the control" of the promoter sequence in a cell when transcription of the coding sequence results from the binding of RNA polymerase to the promoter sequence; translation of the resulting mRNA then results in the polypeptide encoded within the coding sequence.

"Transformation" is the insertion of an exogenous polynucleotide into a host cell. The exogenous polynucleotide may be maintained as a plasmid, or alternatively, may be integrated within the host genome. In cases where the exogenous polynucleotide is RNA, the RNA or a portion thereof will be reverse transcribed into DNA prior to integration.

Two DNA sequences are "substantially homologous" when at least about 90%, and preferably at least about 95%, of the nucleotides match over the defined length of the DNA sequences. Sequences that are substantially homologous can be identified by hybridization experiments, wherein the hybridization is carried out under stringent conditions as defined for that particular system. See, e.g., Maniatis et al., supra; DNA CLONING, Vols. I & II, supra; NUCLEIC ACID HYBRIDIZATION, supra. Detection of non-hybridized regions may be, for example, by S1 nuclease degradation of non-hybridized segments followed by size analysis by gel electrophoresis; or by heteroduplex analysis by electron microscopy.

A "clone" is a population of cells derived from a single cell. A "cell line" is a clone of a primary cell that is capable of stable growth in vitro for many generations.

A "heterologous region" of a polynucleotide construct is an identifiable segment of polynucleotide within the larger polynucleotide molecule that is not found in association with the larger molecule in nature. Thus, when the heterologous region encodes a mammalian gene, the heterologous region will be flanked by a nucleotide sequence that does not flank it in the genome of the source organism. Another example of a heterologous coding sequence is a construct where the coding sequence itself is not found in nature (e.g., a cDNA where the genomic coding sequence contains introns, or a synthetic sequence having one or more codons different than the native gene). Allelic variations or naturally occurring mutational events do not give rise to a heterologous region of DNA.

It is known that all mammals and probably all vertebrates possess basically equivalent MHC systems, and that immune response genes are linked to the MHC. The invention may, therefore, be used to treat autoimmune diseases in vertebrate animals, particularly in mammals. It is expected that the immunotherapeutic method of the invention will be used primarily in humans, as well as in domestic, pet, and sport animals.

Central to the invention is the identification of the alleles within the MHC locus which confer susceptibility to the autoimmune disease of interest, and of the alleles which afford protection against that same autoimmune disease. The presence of these types of alleles is supported by the data correlating the MHC types with diseases. Although there is an association between autoimmune diseases and MHC types, that association is never perfect. While a large fraction of individuals with a disease may all possess the same MHC antigen, the majority of individuals with this antigen do not have the HLA associated disease. For instance, in humans, while more than 90% of individuals with ankylosing spondylitis have B27, only 2-20% of B27 positive individuals in the population have manifestation of ankylosing spondylitis or sacroiilitis. Similarly, it is only a small fraction of DR2 individuals who have multiple sclerosis or of DR3 individuals who have coeliac disease. G. Thomson, HANDBOOK OF EXPERIMENTAL IMMUNOLOGY, Vol. 3: Genetics and Molecular Immunology (D.M. Weir, C. Blackwell, and L.A. Herzenberg eds., 1986) pp. 102.1-102.12.

The present invention relies on two phenomena which underly the lack of disease manifestation in what is thought to be a potentially susceptible individual. First, the genetic locus involved in susceptibility is multi-allelic, and susceptibility is conferred only by certain alleles. An example of this is seen in the case of IDDM in humans. Although there is a strong association of IDDM with DR4, not all individuals with DR4 develop the disease. Based upon restriction length polymorphism analysis (RFLP), the DR4 locus is allelic, and the subtypes 3.1 and 3.2 have been identified. G.T. Nepom (1986) Annals N.Y. Acad. Sci. 475, 1. Individuals who manifest IDDM have the allotype DR4(3.2); individuals who have the allotype DR4(3.1) do not appear to develop the disease. Hence, the DR4(3.2) allele encodes a polypeptide which confers susceptibility to IDDM.

Second, a susceptible individual may not develop the MHC associated autoimmune disease because an allele of a second MHC locus has a protective effect. For example, in the case of IDDM in humans, there is a negative correlation of DR2 with the disease. It has also been suggested, from genetic studies, that DR2 is, or is associated with, a protective factor against the disease. J. Barbosa et al (1979), Tissue Antigens 14, 426; and F. Wolf et a. (1983), Diabetologia 24, 224. In fact, individuals who would be considered susceptible because they carry the DR4 (or DR3) locus who also carry the DR2 locus rarely develop the disease. Nevertheless, some individuals of the haplotype DR4 plus DR2 do develop the disease. The development of the disease in these individuals is explained on the basis of their DR2 subtype. Studies of allelic nature of DR2 by mixed lymphocyte reactivity (MLR) typing indicate that there are several subtypes of DR2. Cohen et al (1984), Proc, Natl. Acad. Sci. U.S.A. 81, 1774. Individuals of the type DR4 (3.2), described above, who do not develop the disease are of the DR2 subtypes DQ1(Dw2) or DQ1(Dw12), while individuals who do develop the disease are of the subtype Mn2 (now identified as similar to DR2/Dw AZH). Wu et al (1986), Nature 324, 676; Lee et al (1987), Proc. Natl. Acad. Sci. U.S.A., in press. Hence, DR2(Dw2) and DR2(Dw12) encode polypeptides which protect the individual from developing IDDM.

A method for identifying which alleles, and subsequently which MHC encoded polypeptides, are associated with an autoimmune disease is the following. First, the association between an MHC antigen and the autoimmune disease is determined based upon genetic studies. The methods for carrying out these studies are known to those skilled in the art (See, E.g., G. Thomson, GENETICS AND MOLECULAR IMMUNOLOGY, supra.), and information on all known HLA disease associations in humans is maintained in the HLA and Disease Registry in Copenhagen. HLA AND DISEASE REGISTRY. THIRD REPORT (Ryder, L.P. Anderson E. & Svejgaard A. eds., 1979) Munksgaard, Copenhagen. The locus encoding the polypeptide associated with the disease is the one that would bear the strongest association with the disease. E.g., based upon the data in Table 2, some of the disease associations in humans would be DR2 for multiple sclerosis, DR4 and/or DR3 for IDDM, DR4 for rheumatoid arthritis, and DR3 for systemic lupus erythramatosus.

Second, specific alleles encoding the disease associated MHC antigen/polypeptide are identified. In the identification of the alleles, it is assumed that the susceptibility allele is dominant. Indentification of the allele is accomplished by determining the strong positive association of a specific subtype with the disease, and of another subtype with lack of the disease. This may be accomplished in a number of ways, all of which are known to those skilled in the art. E.g., subtyping may be accomplished by MLR typing and by primed lymphocyte testing (PLT). HANDBOOK OF EXPERIMENTAL IMMUNOLOGY (D.M. Weir and C.C. Blackwell, ed), supra. (also, reference to the paper doing the Mn2 typing for Dw2). It may also be accomplished by analyzing DNA restriction fragment length polymorphism (RFLP) using DNA probes that are specific for the MHC locus being examined. E.g., G.T. Nepom, Annals N.Y. Acad. Sci., supra. Methods for preparing probes for the MHC loci are known to those skilled in the art. See, e.g., Gregersen et al. (1986), Proc. Natl. Acad. Sci. USA 83, 2642; Wake et al (1982), Nature 300, 372; Stetler et al. (1982), Proc. Natl. Acad. Sci. USA 79, 5966; Weissman et al. in MEDICINE IN TRANSITION: THE CENTENNIAL OF THE UNIVERSITY OF ILLINOIS COLLEGE OF MEDICINE (E.P. Cohen, ed., 1981).

The most complete identification of subtypes conferring disease susceptibility is accomplished by sequencing of genomic DNA of the locus, or cDNA or mRNA encoded within the locus. The DNA which is sequenced includes the section encoding the hypervariable regions of the MHC encoded polypeptide. Techniques for identifying specifically desired DNA with a probe, for amplification of the desired sequences by cloning, and for DNA sequencing are known to those skilled in the art. See. e.g., Maniatis, Fritsch & Sambrook, MOLECULAR CLONING: A LABORATORY MANUAL, supra.; and DNA CLONING, Volumes I and II, supra, for methods in general, and Gregersen et al, supra., for a method particularly applicable to the HLA-DR4 haplotypes in humans. A method for sequencing alleles of DR3 and DR4 which confer susceptibility to IDDM in humans, and of the Aβallele which confers susceptibility to diabetes in Non-Obese Diabetic (NOD) mice is presented in the Experimental Section.

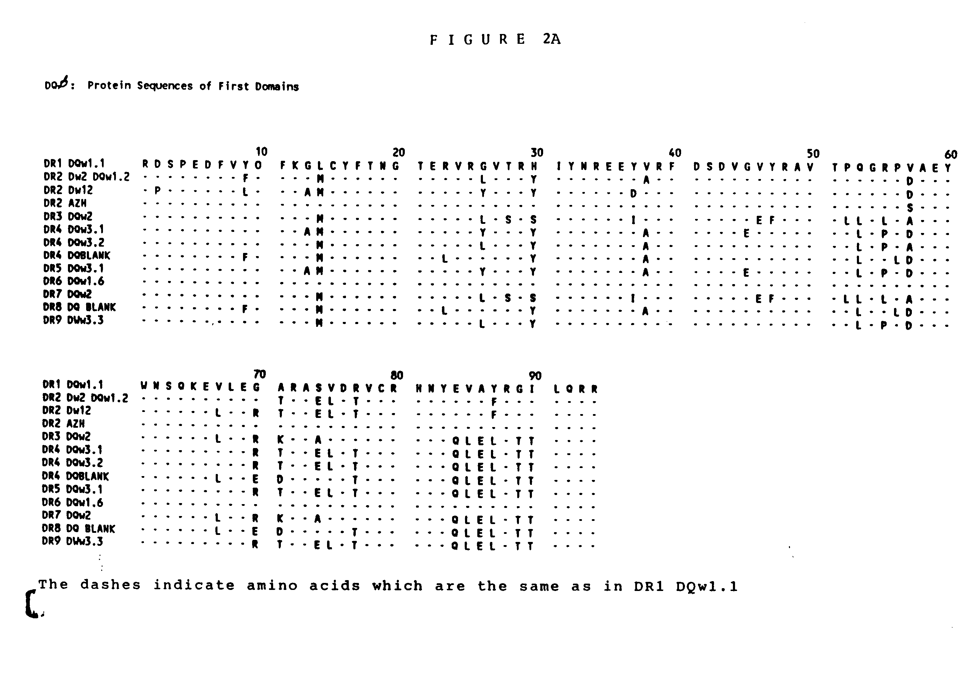

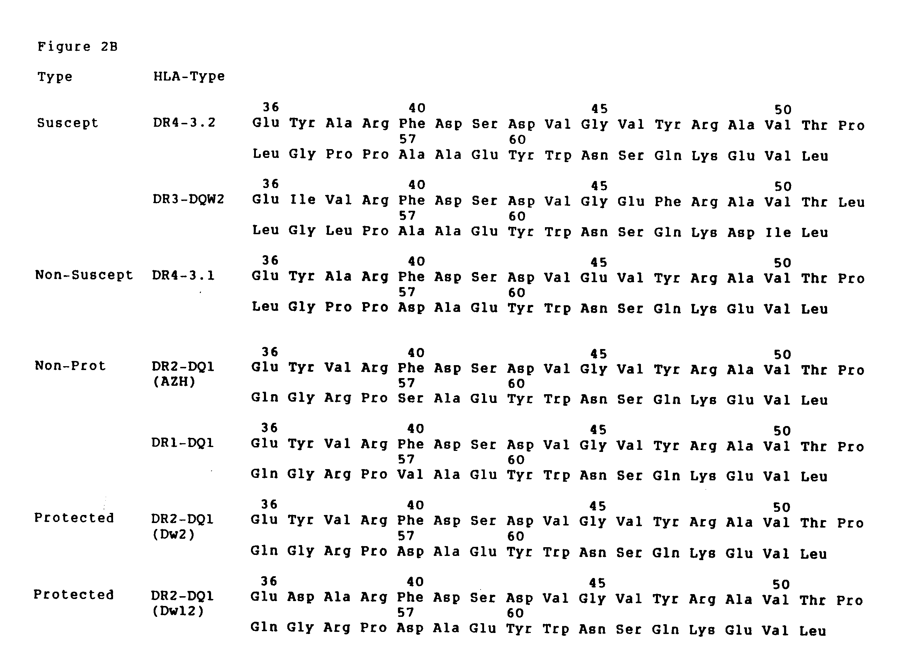

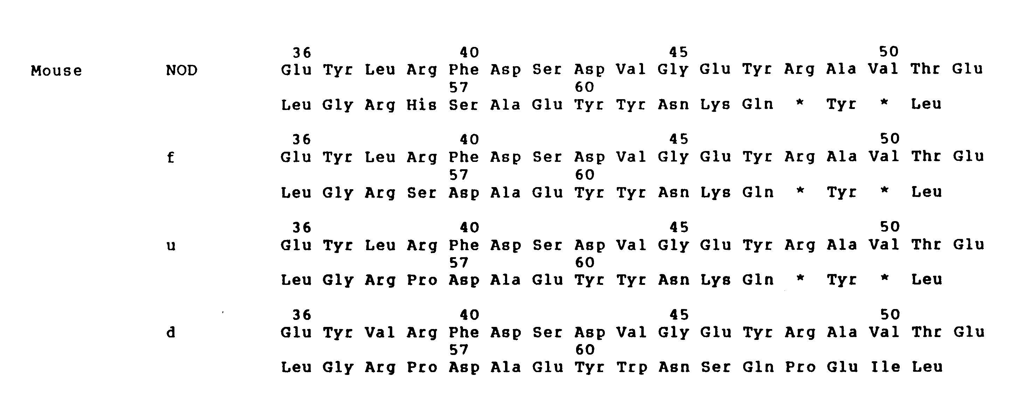

Once the allele which confers susceptibility to the specific autoimmune disease is identified, the polypeptide encoded within the allele is also identifiable. The polypeptide sequence may be deduced from the sequence of the DNA within the allele encoding it. E.g., see Fig. 2 in which the deduced amino acid sequences of regions of the alleles which confer susceptibility to IDDM in humans, and to diabetes in Non-Obese Diabetic (NOD) mice are shown.

A method for identifying a polypeptide which protects against the autoimmune disease, and which is encoded within a second MHC locus is analogous to that described above, for identifying the polypeptide conferring susceptibility. However, the presence of a protective allele is suggested by a negative association of the MHC type which a disease. The subtypes of the MHC type are further examined to determine if specific subtypes, and therefore specific alleles, give a protective effect while other alleles do not. For example, in the case of IDDM, the DR2 type has a negative association with the disease. The DR2 type is subdivided into subtypes, DQ1(Dw2), DQ1(Dw12), and AZH (also called Mn2). Individuals who would be considered "susceptible" to IDDM because they are of the haplotype DR3 and/or DR4(3.2), do not develop IDDM if they also carry either the DQ1(Dw2) or DQ1(Dw12). However, if the DR3 and/or DR4(3.2) individual carries the DR2 subtype AZH, disease incidence is prevalent. Thus, the DQ1(Dw2) and DQ1(Dw12) alleles are protective, while the AZH allele is not protective. Cohen et al (1984), Proc. Natl. Acad. Sci. U.S.A 81, 1774; Setall et al (1986), Human Immunology 17, 61; and Bohme et al (1986), J. Immunology 137 941.

After the alleles conferring protection are identified, they are sequenced, and the polypeptides encoded within them are identified, using the methods described above for the alleles conferring protection.

Subsequent to their identification, the polypeptides encoded within the susceptibility and protective alleles are prepared synthetically. However, prior to synthesis, the fragment of the polypeptide involved in conferring the desired trait is determined, as discussed infra. It is possible that only the hypervariable region of the polypeptide, or a portion thereof, is necessary. In that case, only that portion necessary to confer the trait will be synthesized.

Synthesis may be accomplished by recombinant techniques. Once a sequence encoding a susceptibility or protective MHC antigen has been isolated, it can be cloned into any suitable replicon to create a vector, and thereby be maintained in a composition which is substantially free of vectors that do not contain that sequence. Numerous cloning vectors are known to those of skill in the art, and the selection of an appropriate cloning vector is a matter of choice. See generally, DNA CLONING: VOLS I and II, T. Maniatis et al., supra.

The polynucleotide sequence encoding the MHC antigen is expressed by inserting the sequence into an appropriate replicon, thereby creating an expression vector, and introducing the resulting expression vector into a compatible host.

In creating an expression vector the sequence encoding the MHC antigen is located in the vector with the appropriate control sequences, which include a promoter, a ribosomal binding site, and transcriptional and translational stop codons. The positioning and orientation of the coding sequence with respect to the control sequences is such that the coding sequence is transcribed under the "control" of the control sequences: i.e., the promoter will control the transcription of the mRNA derived from the coding sequence; and the ribosomes will bind at the ribosomal binding site to begin the translational process; and the stop codon used to terminate translation will be upstream from the transcriptional termination codon.

In addition to control sequences, it may be desirable to add regulatory sequences which allow for regulation of the expression of the MHC antigen sequence relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

Modification of the sequences encoding the MHC polypeptide may be desirable. For example, in some cases, it may be necessary to modify the sequence so that it may be attached to the control sequences with the appropriate orientation, i.e. to maintain the reading frame. In some cases, it may be desirable to add sequences which cause the secretion of the polypeptide from the host organism, with subsequent cleavage of the secretory signal. It may also be desirable to produce mutants of the MHC antigen; mutants may be prepared by the deletion of a portion of the sequence encoding the antigen, by insertion of a sequence, and/or by substitution of one or more nucleotides within the sequence. The techniques for modifying nucleotide sequences utilizing cloning are well known to those skilled in the art. They include, e.g., the use of restriction enzymes, of enzymes such as Bal31 to remove excess nucleotides, and of chemically synthesized oligonucleotides for use as adapters, to replace lost nucleotides, and in site directed mutagenesis. See, e.g., Maniatis, Fritsch & Sambrook, MOLECULAR CLONING: A LABORATORY MANUAL (1982); DNA CLONING, Volumes I and II (D.N. Glover, et al ed. 1985); NUCLEIC ACID HYBRIDIZATION (B.D. Hames & S.J. Higgins eds. 1984).

Depending on the expression system and host selected, the MHC peptide is produced by growing host cells transformed by an expression vector described above under conditions whereby the polypeptide is expressed. The synthesized polypeptide is then isolated from the host cells and purified. If the expression system secretes the enzyme into growth media, the receptor protein can be purified directly from the media. If the recombinant polypeptide is not secreted, it is isolated from cell lysates. The selection of the appropriate growth conditions and recovery methods are within the skill of the art.

Isolation of the newly synthesized polypeptide depends upon an assay system by which the polypeptide may be detected. These assay systems would be obvious to one skilled in the art. For example, it is possible to detect the newly synthesized polypeptide based upon its MHC antigenicity. Techniques for identifying MHC antigens/polypeptides are within the skill of the art. See, E.g. HANDBOOK OF EXPERIMENTAL IMMUNOLOGY, supra.

In general, recombinant production of the MHC peptide can provide compositions of that protein which are substantially free of contaminating proteins, i.e. of at least 95% purity. The ability to obtain high levels

EP 0 286 447 B1

of purity is a result of recombinant expression systems which can produce the MHC peptide in substantial quantities vis-a-vis in vivo sources. Thus, by applying conventional techniques to recombinant cultures, MHC peptide compositions of substantial purity and amount are obtainable.

An alternative method to produce the MHC antigen is by chemical synthesis such as solid phase peptide synthesis, using the sequence for the amino acid derived from the DNA sequence of the allele. Such methods are known to those skilled in the art. Chemical synthesis of peptides may be preferable when the desired trait, susceptibility or protection, is conferred by a relatively small fragment of the MHC antigen. Small fragments may be in the range of 60 amino acids or less, preferably in the range of 40 amino acids or less, and more preferably in the range of 20 amino acids or less.

The synthetic MHC peptide or its fragments can be used to produce antibodies, both polyclonal and monoclonal. If polyclonal antibodies are desired, a selected mammal, (e.g., mouse, rabbit, goat, horse, etc.) is immunized with the MHC antigen, or its fragment, or a mutated MHC antigen. Serum from the immunized animal is collected and treated according to known procedures. If serum containing polyclonal antibodies to the MHC peptide contains antibodies to other antigens, the MHC polyclonal antibodies can be purified by immunoaffinity chromatography, using known procedures.

Monoclonal antibodies to the MHC peptide which confers susceptibility to a specific autoimmune disease, as well as to the MHC peptide which confers protection against that autoimmune disease, and to the fragments thereof, which contain the epitope(s) necessary for either susceptibility or protection, can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., M. Schreier et al., HYBRIDOMA TECHNIQUES (1980); Hammerling et al., MONOCLONAL ANTIBODIES AND T-CELL HYBRIDOMAS (1981); Kennett et al., MONOCLONAL ANTIBODIES (1980); see also U.S. Patent Nos. 4,341,761; 4,399,121; 4,427,783; 4,444,887; 4,452,570; 4,466,917; 4,472,500, 4,491,632; and 4,493,890. Monoclonal antibodies to MHC antigens are known to those skilled in the art. See, e.g., Weyand et al. (1986), Proc. Natl. Acad. Sci. USA 83, 762. Panels of monoclonal antibodies produced against the MHC antigen or fragment can be screened for various properties; i.e., for isotype, epitope, affinity, etc. Monoclonal antibodies directed against specific epitopes are useful in defining interactions of which confer susceptibility or resistance. Monoclonal antibodies are also are useful in purification, using immunoaffinity techniques, of the MHC peptides which they are directed against. In addition, as discussed below, monoclonal antibodies may be used in determining which individuals are susceptible, and which individuals are protected from that susceptibility.

In the preparation of antibodies, prior to immunization, it may be desirable to increase the immunogenicity of the MHC peptide, or a mutant of the MHC peptide, or particularly fragments of the MHC peptide. This can be accomplished in any one of several ways known to those of skill in the art. For example, the antigenic peptide may be administered linked to a carrier. For example, a fragment may be conjugated with a macromolecular carrier. Suitable carriers are typically large, slowly metabolized macromolecules such as: proteins; polysaccharides, such as latex functionalized sepharose, agarose, cellulose, cellulose beads and the like; polymeric amino acids such as polyglutamic acid, polylysine, and the like; amino acid copolymers; and inactive virus particles. Especially useful protein substrates are serum albumins, keyhole limpet hemocyanin, immunoglobulin molecules, thyroglobulin, ovalbumin, and other proteins well known to those skilled in the art.

The protein substrates may be used in their native form or their functional group content may be modified by, for example, succinylation of lysine residues or reaction with Cys-thiolactone. A sulfhydryl group may also be incorporated into the carrier (or antigen) by, for example, reaction of amino functions with 2-iminothiolane or the N-hydroxysuccinimide ester of 3-(4-dithiopyridyl) proprionate. Suitable carriers may also be modified to incorporate spacer arms (such as hexamethylene diamine or other bifunctional molecules of similar size) for attachment of peptides.

Individuals who are susceptible to the autoimmune disease, an who do not carry the protective allele are protected against the disease by tolerizing them to the self-antigen involved in the autoimmune response. This antigen is probably not encoded within the MHC locus, but bears an epitope with a structural resemblance to the epitope contained within the MHC peptide which affords protection. Hence, individuals who carry the protective sequence in addition to the susceptibility sequence are normally tolerant to the antigen and do not develop the disease. However, individuals who are not normally tolerant, but who are susceptible may be tolerized using the MHC peptide, or protective fragment thereof, or mutant thereof, carrying the epitope encoded within the protective MHC allele. This is shown further in the Experimental section, using the animal model for IDDM, the Non-obese Diabetic Mouse (NOD).

An individual may be tolerized to the MHC peptide affording protection, or to a protective fragment thereof, or a protective mutant thereof, in any one of several ways known to those of skill in the art. For example, the protective peptide, or protective fragment thereof, or mutant thereof, may be administered linked to a carrier. For example, the MHC protective antigen may be conjugated with a macromolecular carrier. Methods for conjugating antigens to carriers, as well as suitable carriers, have been described supra.

Especially suitable carriers for the MHC protective peptide/fragment/mutant are cells, and particularly lymphocytes, since presentation in this form mimics the natural mode of presentation in the individual, which gives rise to the tolerizing effect. Therefore, the peptide may be covalently coupled to lymphocytes, preferably the individual's own peripheral lymphocytes. There are injected parenterally, and preferably intravenously. It has been demonstrated that peptides or antigenic determinants coupled to spleen cells identical to the host's own spleen lymphocytes, when injected intravenously, induce immunologic suppression against the coupled peptide or protein. Sriram et al (1983), Cell. Immunol. 75, 378; McKenna et al (1983) Cell. Immunol. 81, 391; Cheung et al (1981), Exp. Med. 148, 1539, and Schoen et al (1982), J. Immunol. 128, 717. Methods of coupling peptides to proteins or cells are known to those of skill in the art, and one such method is discussed in the Experimental section.

Alternatively, the MHC antigen affording protection, or a protective fragment thereof, or mutant thereof, may be coupled to erythrocytes, preferably the individual's own erythrocytes. The techniques for coupling to erythrocytes are essentially the same as those used for coupling to lymphocytes. McKenna et al (1983), supra.

It is also possible to tolerize an individual with the MHC peptide affording protection, or a protective fragment thereof, or a mutant thereof, which is not coupled to a carrier, but which is administered neat, or mixed with a pharmaceutically acceptable adjuvant, carrier or diluent. Liquid pharmaceutically administerable compositions particularly for parenteral administration (generally characterized by injection - subcutaneously, intramuscularly, or intravenously) can be prepared by dissolving, dispersing, etc. the polypeptide of the invention and optional pharmaceutical adjuvants in a carrier, such as, for example, water, saline, aqueous dextrose, glycerol, ethanol, and the like, to thereby form a solution of suspension. If desired, the pharmaceutical composition to be administered may also contain minor amounts of nontoxic auxiliary substances such as wetting or emulsifying agents, pH buffering agents and the like. Actual methods of preparing such dosage forms are known, or will be apparent, to those skilled in the art. See, for e.g., REMINGTON'S PHARMACEUTICAL SCIENCES, Mack Publishing Company, Easton, Pennsylvania, 15th Edition, 1975. The composition or formulation to be administered will, in any event, contain a quantity of the protective peptide/polypeptide adequate to achieve the desired tolerized effect in the individual being treated.

The individual is tolerized by administration of the protective peptide encoded within the allele, or a protective fragment thereof, or a mutant thereof. If the fragment or mutant is used, it will include the amino acid sequence of the epitope which interacts with the immune system to tolerize the animal to that and structurally similar epitopes. The sequence for the epitope will be within the hypervariable region of the MHC encoded polypeptide. An example of this with respect to IDDM is shown in Figure 2, and is discussed in the Experimental section.

As shown in the Experimental section, in the case of protection against IDDM, it appears that the the amino acids at position 56 and 57 of the $\beta$-chain of DR2 form part of the epitope which causes tolerance in protected individuals. This was ascertained for humans by sequencing the DR2 protective alleles, the DR2 non-protective allele, and the DR4 susceptible and non-susceptible alleles, deducing the amino acid sequences from the nucleotide sequences, and comparing the deduced amino acid sequences of the alleles. This comparison showed that the susceptible individuals all had neutral amino acids at position 57 of the DR4 $\beta$-chain; in comparison, all non-susceptible individuals had aspartate, an acidic amino acid, at position 57. All protected individuals also had the acidic amino acid, aspartate, at positions 57 of the DR2 $\beta$-chain. This strongly suggests that the amino acid at position 57 is part of an epitope which is involved in protection against IDDM. Moreover, this suggestion is supported by the results obtained with Non-Obese Diabetic (NOD) mice, which are an animal model for human IDDM. In NOD mice, residue 57 of the A$\beta$ chain is the neutral amino acid, serine; in comparison, the A$\beta$ chain of mice that do not develop diabetes has aspartate at residue 57 (See Fig. 2). Thus, protective fragments administered to protect humans against IDDM would most likely contain the amino acid sequence forming the epitope in the HLA DR2 $\beta$-chain which includes aspartate at amino acid residue 57.

Protective fragments used to tolerize an individual contain at least 9 amino acids which form the sequence of the protective epitope, more preferably contain 20 amino acids including the protective epitope, and may extend to contain the entire hypervariable region containing the epitope. Smaller fragments encompassing the epitope may be inserted into larger peptides or polypeptides, such that the regions flanking the epitope are not those that are encoded within the naturally occurring MHC protective allele; the resulting polypeptides are mutants of the protective MHC antigen. The techniques for the synthesis of these peptides or polypeptides are apparent to one of average skill in the art. For example, the genetic sequence encoding a protective MHC antigen may be isolated via cloning, and that sequence altered at sites other than that encoding the protective epitope. This alteration may be accomplished by site specific mutation, or by deletions, or by insertions. Alternatively, an oligonucleotide sequence encoding the epitope may be inserted into or attached to another sequence which encodes a different peptide or polypeptide. A recombinant sequence is then inserted into an expression vector which is compatible with the host to be transformed, and the expression system used to synthesize the desired peptide which includes the protective epitope. The techniques by which this may be accomplished are known to those of skill in the art. See, e.g. Maniatis, Fritsch & Sambrook, MOLECULAR CLONING; A LABORATORY MANUAL, supra; DNA CLONING, Volumes I and II, supra; and NUCLEIC ACID HYBRIDIZATION, supra. Alternatively, an oligopeptide may be synthesized by solid phase synthesis which includes the protective epitope, but which adds flanking amino acids to it which are not in the sequence of the naturally occurring antigen.

To tolerize an individual, the protective MHC-antigen, or the protective fragment thereof, is administered parenterally, usually by intravenous injection, however other modes of administration which cause the tolerized state are also acceptable, e.g. oral administration. The dose given is that which will cause the tolerized state in the individual, and is usually 1 $\mu$g to 10 mg per gram, and is preferably 10 $\mu$g to 1 mg per gram, and is more preferably 50$\mu$g to 500 $\mu$g per gram. The individual is tolerized by administration of the protective antigen or protective fragment thereof, or protective mutant thereof, in at least one dose, and preferably two doses. Moreover, the individual may be administered as many doses as is required to maintain a state of tolerance to the self-antigen. The present of the tolerized state is monitored. Methods of monitoring the tolerized state are known to those of skill in the art. See, for e.g., Gammon et al (1986), Nature 319, 413. Moreover, since the effects of many autoimmune diseases are considered irreversible, e.g. destruction of the insulin producing islets of the pancreas during IDDM, tolerization of the susceptible individual will be prior to full manifestation of the disease, and preferably prior to the onset of the disease.

Procedures for tolerizing NOD mice is presented in the Experimental section. The Non-obese Diabetic Mouse (NOD) provides an excellent murine model for IDDM in humans. Wicker et al (1986), Diabetes 35, 855. As in humans, susceptibility in these mice to diabetes maps to the major histocompatibility complex, as well as to other non-MHC linked genes.

The present invention also encompasses diagnosing individuals who are susceptible to an autoimmune disease before manifestation of the disease, since these individuals will benefit from the above described tolerization procedure. Diagnosis is accomplished by determining an individual's susceptibility to the disease based on the presence of the MHC antigen conferring susceptibility, and the absence of the MHC antigen conferring protection.

A method for identifying the alleles conferring susceptibility to an autoimmune disease, and protection from it, has been described above. Once these alleles have been identified and sequenced, many techniques are available for diagnosis of individuals.

First, the individual may be screened with respect to MHC to determine whether it falls within the potentially susceptible class. MHC typing may be by serologic (DR typing in humans) and/or MLC (Dw typing in humans). Methods for MHC typing are well known in the art. E.g., HANDBOOK OF EXPERIMENTAL IMMUNOLOGY, Vol 3: Genetics and Molecular Immunology (D.M. Weir, C. Blackwell, and L.A. Herzenberg, eds., 1986).

An alternative to serologic and MLC typing to determine potentially susceptible individuals are methods which utilize nucleic acid hybridization techniques. These methods, which utilize oligonucleotide probes specific to the variant region within the allele in combination with RFLP, distinguish between the allelic subtypes, and are known to those of skill in the art. See, e.g., Bell et al (1986), Lancet, May issue, p 1058, Wake et al (1982), supra., and Owerbach et al (1983), Nature 303, 815.

Potentially susceptible individuals may also be determined using methods which utilize monoclonal antibodies specific to epitopes unique to the antigens encoded within the alleles conferring susceptibility. Methods for producing these monoclonal antibodies have been discussed above. The presence of the antigens, and thus the susceptible allotypes, is determined by an assay that indicates whether monoclonal test antibodies selectively bind these antigens in a test sample. Various binding assay techniques are known in the art. Some of these techniques are described in U.S. Patent Number 4,607,009, issued Aug. 19, 1986, which is incorporated herein by reference. Generally, these assays involve "tagging" either the monoclonal test antibody or an antibody that selectively binds the monoclonal test antibody (indicator antibodies). The test sample is then either (a) contacted with tagged test monoclonal antibodies, or (b) contacted first by untagged monoclonal test antibodies followed by contact with tagged indicator antibodies. Detection of the tag associated with the test sample indicates that monoclonal test antibodies have selectively bound an antigenic determinant in the test sample.

Potentially susceptible individuals may be tolerized with the protective antigen as described above, or preferably they are further screened to determine the presence or absence of the MHC allele(s) conferring protection. A method for identification of these protective alleles has been described above. The presence or absence of the protective alleles is determined using methods analogous to those described above for determining the presence or absence of the susceptibility alleles, except that the probes used are specific for the susceptibility alleles. Individuals who have the susceptibility allele and lack the protective allele are considered extremely susceptible to the autoimmune disease.

The various embodiments of the present invention are useful in several respects. First, the purified MHC antigen, or protective fragment thereof, which is encoded within the allele conferring protection against an autoimmune disease, may be used as prophylactic treatment for that autoimmune disease by methods which tolerize the individual to that antigen. At present there are a variety of autoimmune diseases of humans for which this treatment might be efficacious, including IDDM, juvenile rheumatoid arthritis, ankylosing spondylitis, rheumatic fever, Addison's disease, and multiple sclerosis, and including the other autoimmune diseases listed in Tables 1 and 2.

The identified allelic sequences which encode the protective and susceptibility antigens for an autoimmune disease may be used for the synthesis of oligonucleotide probes. These probes are useful in the diagnosis of individuals who are highly susceptible to that autoimmune disease.

Monoclonal antibodies to the unique epitopes of the identified susceptibility and protective antigens are useful for diagnosis of susceptible individuals. In addition, monoclonal antibodies to the susceptibility antigens are useful in defining how these self-antigens interact with other antigens to give rise to the autoimmune disease. Also, monoclonal antibodies to the protective antigens are useful in defining the non-MHC antigen involved in the autoimmune disease.

Described below are examples of the present invention which are provided only for illustrative purpose, and not to limit the scope of the present invention. In light of the present disclosure, numerous embodiments within the scope of the claims will be apparent to those of ordinary skill in the art.

Experimental

1. Materials and Methods

Enzymes were purchased from commercial sources, and used according to the manufacturers' directions. Radionucleotides and nitrocellulose filters were also purchased from commercial sources.

DNA was sequenced by the dideoxynucleotide chain termination method in bacteriophage m13. Sanger et al (1977), Proc. Natl. Acad. Sci. USA 74, 5463.

DNA oligomers were synthesized by automated oligomer synthesis, according to the manufacturer's instructions, using the Applied Biosystem Peptide Synthesis System.

In the cloning of DNA fragments, except where noted, all DNA manipulations were done according to standard procedures. See, Maniatis et al., MOLECULAR CLONING, supra. Restriction enzymes, $T_4$ DNA ligase, E. coli, DNA polymerase I, Klenow fragment, and other biological reagents were purchased from commercial suppliers and using according to the manufacturers' directions. Double-stranded DNA fragments were separated on agarose gels.

cDNA libraries were prepared by standard techniques in λgt10. See, T.V. Huynh et al., in DNA CLONING: VOL 1 (Glover, Ed.), supra.

Amplified preparations of cDNA from RNA were prepared as follows. In the procedure, all materials and solutions used in the preparation of RNA were RNase free, and water was doubly distilled and treated with diethyl pyrocarbonate. RNA was isolated using the guanidiniumthyocyanate method. Chirgwin, J.M. et al (1979), Biochemistry 18, 5294, see also, Maniatis et al, MOLECULAR CLONING: A LABORATORY MANUAL, supra.. RNA was isolated from PBLs, which were separated and harvested from 50 - 100 ml of fresh blood; alternatively RNA was isolated from tissue culture cells harvested in exponential growth, or from the tissue and organs of freshly killed animals. Specific cDNAs were amplified and cloned using essentially the technique described by Saiki et al, and Scharf et al., which are hereby incorporated by reference. Saiki et al (1986), Nature 324, 163; and Scharf et al (1986), Science 233, 1076. MHC typing, and particular HLA typing and subtyping were performed using standard techniques known to those skilled in the art. See, e.g. HANDBOOK OF EXPERIMENTAL IMMUNOLOGY, Volumes I-IV, supra.

## 2. Sequencing of Alleles Conferring Susceptibility to IDDM.

Human subjects selected were classical IDDM patients, i.e. the age of disease onset was less than 20 years; subjects were chosen by random selection.

The techniques used for isolating PBL were standard, see, for e.g., THE HANDBOOK OF EXPERIMENTAL IMMUNOLOGY, supra..

The cDNAs encoding DQ$\beta$ 3.1 and DQ$\beta$ 3.2 were obtained from total RNAs, and amplified cDNAs which were cloned, using the above described techniques. The cDNAs were sequenced after cloning into M13mp10. The cloned cDNAs were sequenced directly; further probing was unnecessary.

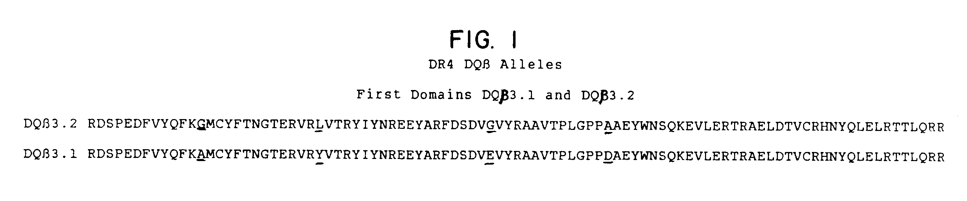

A comparison of the deduced amino acid sequences for the first domains of two allelic DR4 antigens/polypeptides, DQ$\beta$ 3.1 and DQ$\beta$ 3.2, are presented in Fig. 1. In this figure the amino acids are represented by alphabetic code; the differing amino acids are underlined. It may be seen that the two amino acid sequences differ by only four amino acids. At least 90% of diabetics are DR3 or DR4, and 95% of the DQ$\beta$ genes on the DR4 haplotype in diabetics are of the DQ$\beta$ 3.1 allelic subtype. This allele is present with a 65% frequency in healthy individuals. On the other hand, only 5% of diabetics with DR4 have the DQ$\beta$ 3.1 allele compared to 35% of healthy DR4 individuals. The DQ molecule is comprised of two chains, $\alpha$ and $\beta$. The $\alpha$ chain is identical in both chromosomes carrying the 3.1 and 3.2 alleles. Hence, the susceptibility conferred on the patient by the DQ$\beta$ 3.2 allele must be associated with any or all of these four residues which differ between the two chains. There is no other subtype or different allele of the same gene, for example, DR$\beta$, that is markedly enriched in diabetics compared to healthy subjects.

The deduced amino acid sequences of residues of the first domain of the $\beta$-chain for the DR3 and DR4 allelic antigens/polypeptides linked to susceptibility are presented in Fig. 2A. A consistent feature of these sequences is the present of a neutral amino acid at residue 57 of the first domain of the $\beta$-chain.

## 3. Sequencing of Alleles Conferring Protection to IDDM.

Subjects were selected randomly.

The cDNAs and genomic DNAs were isolated and amplified as described supra.

The deduced amino acid sequences of the first domain of the $\beta$-chain for DR2 allelic antigens/polypeptides conferring protection are presented in Fig. 2A; A comparison of residues 36-68 of sequences conferring protection to those conferring susceptibility is shown in Fig. 2B. A comparison of these sequences to the analagous allelic sequences which do not confer protection are shown in the same Figure. The fact that all of the sequences which protect contain the acidic residue aspartic acid at position 57, while the non-protective sequences and the sequences conferring susceptibility all contain neutral amino acids at this position, raises the possibility that the nature of the residue at this position, i.e., acidic or neutral, may be of importance in determining susceptibility vs. protection with respect to IDDM.

## 4. Sequencing of Alleles Conferring Susceptibility and Non-susceptibility to Diabetes in Mice.

The murine analog of DQ$\beta$, A$\beta$, was sequenced; RNA was isolated, and cDNA libraries prepared as discussed above. More particularly, total RNA was used for the protection of cDNA. After the second strand RNase H-Polymerase I reaction, standard methods were used to obtain blunt ended molecules, to protect internal EcoRI sites with EcoRI methylase, and T$_4$ DNA ligase was used for ligating phosphorylated EcoRI linkers. By cutting with EcoRI, EcoRI sites were created at both ends of the molecules and the cDNA was separated from the cut linker molecules by electrophoresis in a 1% agarose gel. The cDNA molecules above 700 base pairs were electroeluted onto DEAE membranes (Schleicher and Schuell. DEAE membranes, NA45). After washing in water and low salt, more than 80% of the bound material could be eluted within 15 minutes at 65°C in 1.5 M NaCl, 20 mM Tris, 5 mM EDTA at pH 7.5. The cDNA molecules were ligated in EcoRI cut $\gamma$gt10, packaged in vitro and screened using standard methods. By routinely using 50 $\mu$g of total RNA as starting material, more than $10^6$ independent clones could be isolated. Because more than 95% of the total RNA is ribosomal RNA, the library was screened with a 6.2 kb EcoRI genomic DNA fragment encoding part of the 18S and the 28S mRNA. Tiemeier, D.C. et al (1977). Gene 2, 173. This allowed an estimate of the percentage of cDNA clones obtained from oligo dT-priming in the first strand reaction at sites other than poly(A) tails. Less than 5% of the clones hybridized to this probe. The frequency of I-A$_\alpha$ clones was 1/2000 and for I-A$_\beta$, 1/3000. More than 90% of the isolated clones contained 5' as well as 3' untranslated sequences.

The library was screened with an A$_\alpha^k$ cDNA probe and an A$_\beta^k$ probe at a density of 10,000 plaque forming units per 150 mm plate as described in Huynh, T.V. (1984) in DNA CLONING TECHNIQUES: A PRACTICAL APPROACH, ed. D. Glover (IRL Press), pp 49-78. The $A_\alpha^k$ probe and $A_\beta^k$ probe were prepared according to Benoist et al., and Estess et al., respectively. Benoist et al (1983). Proc. Natl. Acad. Sci. U.S.A. 80, 534; Estess. P., et al.(1986), Proc. Natl. Acad. Sci. U.S.A. 83, 3594.

The deduced sequence of amino acid residues 36-68 of the first domain of the $A\beta$ region of the NOD mouse is shown in Fig. 2B. Fig. 2B also shows the corresponding deduced amino acid sequences for the $A\beta$ region of three non-susceptible mice, f, u and d. Similar to the finding in humans, residue 57 in the NOD mouse contains a neutral amino acid, serine, while the non-susceptible mice have the acidic amino acid, aspartate, at that position. These results are consistent with the suggestion that the epitope containing residue 57 is centrally involved in susceptibility to diabetes.

5. Prevention of Diabetes in NOD by Induction of Cross Tolerance to MHC Class II Peptides

The experiment is based on the premise that immunoreactive peptides in foreign proteins bear some structural homology to self antigens, which include the MHC class II molecules. The NOD MHC allele $A\beta$ confers disease susceptibility by virtue of its unique amino acid sequence at positions 56 and 57 of the protein. These critical amino acids form an epitope in the $A\beta$ chain that is immunologically non-cross reactive with an epitope in the putative pancreatic antigen. This lack of tolerance permits the animal to mount an immune response against the pancreatic antigen leading to destruction of the insulin-producing $\beta$ cells.

In healthy animals, carrying other $A\beta$ genes with the protective amino acids at residues 56 and 57, the $A\beta$ chain peptide structure is so similar to the structure of the critical epitope of the pancreatic antigen that normal tolerance is achieved. Therefore, NOD mice are protected by tolerizing them with a peptide containing the protective amino acids.

Tolerization of NOD mice is accomplished with a peptide which is a 20-mer, and which corresponds to the sequence from residue 40-60 of the human DR2 protective allele. This 20-mer has the sequence N-Cys-Asp-Ser-Asp-Val-Gly-Val-Tyr-Arg-Ala-Val-Thr-Pro-Gln-Gly-Arg-Pro-Asp-Ala-Glu-Tyr-C. The human DR2 allele is almost identical to the protective mouse allele sequence.

Adult female mice are tolerized using the peptide coupled to spleen cells. The peptide is covalently cross-linked to NOD spleen cells via the N-terminal cystine residue using the heterobifunctional photoreactive reagent, p-Azidophenylacyl bromide, which is commercially available. The conditions for cross-linking are essentially as described in Moreland et al. (1981), Biochem. Biophys. Res. Comm. 99, 339. More specifically, 500$\mu$g peptide are linked to the photoreactive agent in the dark. 5ml of cells, at $10^7$ cells/ml, of twice washed single spleen cells, freshly removed from 3-4 week old NOD mice are reacted with the peptide linked to the photoreactive agent, using 100$\mu$g/ml peptide. The reaction is carried out in the dark, at 37°C for 30 min. The cells are then washed extensively in PBS, and after washing, subjected to U.V. light to cross-link the activated peptide onto the surface proteins of the cells. These cells are then washed twice in PBS and resuspended to $10^8$ cells/ml. Each mouse is injected intravenously with 0.5ml of the resuspended cells. The first injection is at 2 weeks after birth; this is followed by a second injection at 4 weeks after birth.

Control NOD adult female mice are tolerized with a peptide corresponding to the NOD sequence, N-Cys-Asp-Ser-Asp-Val-Gly-Glu-Tyr-Arg-Ala-Val-Thr-Glu-Leu-Gly-Arg-His-Ser-Ala-Glu-Tyr-C, coupled to spleen cells. The coupling procedure and injection procedure are identical to that described for the part of the experiment with the protective sequence.

Neonatal tolerance of female NOD mice is induced by injecting mice with $1 \times 10^7$ to $5 \times 10^7$ peptide-coupled spleen cells within 24 hours of birth; the second injection, using the same amount of cells, is given three days after the first injection.

Neonatal tolerance of female mice is also accomplished by induction with purified peptide using known techniques. Gammon et al (1986), Nature 319, 413. More specifically, purified peptide, i.e., the DR2 peptide for the tolerized mice, the mouse "protective" residue, and the NOD peptide for the control mice, is dissolved in PBS and emulsified in complete Freunds adjuvant. The mice are injected with 20$\mu$g of peptide. The injection is into the peritoneal cavity, and is given at 24 hours after birth, and again at 72 hours after birth.

After 3 months, the animals are monitored for urine levels of glucose to measure disease onset. The animals used are all female, because in female NOD mice disease incidence is, by 30 weeks of age, approximately 72%. The incidence of disease in male mice at this age is only 39%. See, Wicker et al (1986), supra. Each group of animals consists of at least 20 mice, and each litter is split in two halves. One half is used for the DR2 protective peptide tolerization, and the other half for the control NOD peptide injection.

## 6. Determining Whether an Individual Should be Tolerized with a Protective MHC Polypeptide Offering Protection against IDDM

Offspring of an IDDM parent are potential subjects for tolerization. Further screening is by genotyping the individual using dot blot analysis for the HLA genes conferring susceptibility, and for the protective alleles. Dot blot analysis is by known procedures, see e.g. Saiki et al, supra, using as probes oligonucleotides which hybridize to the variable regions of the first domain of the following alleles: DR4 3.2, DR3 Dqw2, DR4 3.1 DR2 DQ1, DR1 DQ1, DR2 DQ1 (Dw2) and DR2 DQ1 (DW12). Individuals of the genotype DR4 3.2 or DR3 DQw2, who are potentially susceptible,. and who lack DR2 DQ1 (Dw2) or DR2 DQ1 (Dw12) will be tolerized with the protective polypeptide which tolerizes against IDDM.

Screening may also be accomplished by the detection of antibodies or antigens which appear prior to the clinical onset of IDDM. These antibodies are a 64.000 $M_r$ human islet cell antigen, the islet cell cytoplasmic antibodies (ICCA), and the islet cell surface antibodies (ICSA). Baekkeskov, S. (1987), J. Clin. Invest. 79, 926. These antibodies and antigens may be detected using an ELISA assay which utilizes monoclonal antibodies to the specific antibodies and antigens. Individuals who exhibit these antibodies and/or antigen will be tolerized as discussed above.

While the present invention has been illustrated above by certain specific embodiments, it is not intended that these specific examples limit the scope of the invention as described in the appended claims.

For example, the invention is not limited to prophylactic treatment against only IDDM, but may extend in its various aspects to other human autoimmune diseases for which susceptibility is associated with a relevant first HLA type allele (such as set out in Tables 1 and 2 above) e.g. those associated with HLA-D and HLA-DR antigens.

## Claims

1. A pharmaceutical composition for prophylactically treating an individual for an autoimmune disease to which the individual is susceptible, the autoimmune disease being one to which susceptibility is associated with a first MHC type allele, and to which non-susceptibility is associated with a second MHC type allele which is protective against the disease, the pharmaceutical composition comprising a tolerizing amount of an antigen encoded within the protective allele, wherein the antigen causes the protective effect.

2. A pharmaceutical composition for prophylactically treating a human for IDDM to which the human is susceptible, the susceptibility being associated with a first HLA type allele, and wherein a second HLA allele is protective against IDDM, the pharmaceutical composition comprising a tolerizing amount of an antigen encoded within the protective HLA allele, wherein the antigen causes the protective effect.

3. The composition of claim 2, wherein the protective antigen is encoded within DR2 DQ1 (Dw2) or DR2 DQ1 (Dw12) or DR4 DQw 3.1.

4. The composition of claim 2, wherein the antigen is a protective fragment or a mutant of a protective antigen encoded within the protective HLA allele, wherein said protective fragment or said mutant contains an amino acid sequence which forms an epitope which is strongly cross-reactive with that epitope in the protective HLA sequence which gives rise to the protective effect.

5. The composition of claim 2, claim 3 or claim 4, wherein the antigen is a polypeptide comprised of the amino acid sequence

```
N-pro-gln-gly-arg-pro-asp-ala-glu-tyr-C
```

or

```
N-pro-leu-gly-pro-pro-asp-ala-glu-tyr-C
```

or

```
N-pro-gln-gly-arg-ser-asp-ala-glu-tyr-C,
```

wherein said polypeptide contains an amino acid sequence which forms an epitope which is strongly

17

cross-reactive with that epitope in the protective HLA sequence which gives rise to the protective effect.

6. A method for determining whether an individual should be treated prophylactically for an autoimmune disease, the autoimmune disease being one to which susceptibility is associated with a first MHC type allele, and to which non-susceptibility is associated with a second MHC type allele which is protective against the disease, the method comprising determining whether an individual has the allele conferring susceptibility, and being characterized in, for said potentially susceptible individuals, determining whether a protective allele is absent.

7. The method of claim 6, wherein the autoimmune disease is IDDM, the individual is human, and it is determined whether the individual has the allele conferring susceptibility, the alleles conferring susceptibility comprising DR3 and DR4(3.2), and whether the individual lacks an allele conferring protection, the alleles conferring protection comprising DR2 DQ1(Dw2) and DR2 DQ1(Dw12).

8. A method for preparing a polypeptide containing a protective epitope against an autoimmune disease in an individual to which the individual is susceptible, the autoimmune disease being one to which susceptibility is associated with a first MHC allele, characterized in identifying a second MHC type allele which is protective against the disease, and synthesizing the polypeptide by chemical means, using the amino acid sequence for the epitope derived from the DNA sequence of the protective allele.

9. A method for preparing a polypeptide containing a protective epitope against an autoimmune disease in an individual to which the individual is susceptible, the autoimmune disease being one to which susceptibility is associated with a first MHC allele, characterized in identifying a second MHC type allele which is protective against the disease, and expressing a recombinant vector encoding said polypeptide in a compatible host, and isolating said polypeptide.

10. The method of claim 8 or claim 9, wherein the individual is human, the autoimmune disease is IDDM, and wherein the amino acid sequence of the protective epitope is encoded within DR2 DQ1 (Dw2) or DR2 DQ1 (Dw12) or DR4 DQw 3.1.

**Patentansprüche**

1. Pharmazeutische Zusammensetzung zur prophylaktischen Behandlung eines Individuums gegen eine Autoimmunerkrankung, für die das Individuum anfällig ist, wobei die Autoimmunerkrankung eine ist, bei der die Anfälligkeit dafür mit einem ersten MHC-Typ-Allel assoziiert ist, und bei der die Nicht-Anfälligkeit dafür mit einem zweiten MHC-Typ-Allel assoziiert ist, das gegen die Erkrankung schützt, wobei die pharmazeutische Zusammensetzung eine Toleranzmenge eines Antigens umfaßt, für das innerhalb des Schutzallels kodiert wird, worin das Antigen die Schutzwirkung bewirkt.

2. Pharmazeutische Zusammensetzung zur prophylaktischen Behandlung eines Menschen auf IDDM, für die der Mensch anfällig ist, wobei die Anfälligkeit mit einem ersten HLA-Typ-Allel assoziiert ist, und worin ein zweites HLA-Allel gegen IDDM schützt, wobei die pharmazeutische Zusammensetzung eine Toleranzmenge eines Antigens umfaßt, für das innerhalb des schützenden HLA-Allels kodiert wird, worin das Antigen die Schutzwirkung bewirkt.

3. Zusammensetzung nach Anspruch 2, worin innerhalb von DR2 DQ1 (Dw2) oder DR2 DQ1 (Dw12) oder DR4 DQw 3.1 für das schützende Antigen kodiert wird.

4. Zusammensetzung nach Anspruch 2, worin das Antigen ein Schutzfragment oder ein Mutant eines schützenden Antigens ist, für das innerhalb des schützenden HLA-Allels kodiert wird, worin das genannte Schutzfragment oder der genannte Mutant eine Aminosäuresequenz enthält, die ein Epitop bildet, das mit jenem Epitop in der schützenden HLA-Sequenz stark über Kreuz reagiert, das die Schutzwirkung auslöst.

5. Zusammensetzung nach Anspruch 2, 3 oder 4, worin das Antigen ein Polypeptid ist, das die Aminosäuresequenz

```
N-pro-gln-gly-arg-pro-asp-ala-glu-tyr-C
                    oder
N-pro-leu-gly-pro-pro-asp-ala-glu-tyr-C
                    ·oder
N-pro-gln-gly-arg-ser-asp-ala-glu-tyr-C
```

enthält, worin das genannte Polypeptid eine Aminosäuresequenz enthält, die ein Epitop bildet, das mit dem Epitop in der schützenden HLA-Sequenz stark über Kreuz reagiert, das die Schutzwirkung auslöst.

6.  Verfahren, um zu bestimmen, ob ein Individuum prophylaktisch gegen eine Autoimmunerkrankung behandelt werden sollte, wobei die Autoimmunerkrankung eine ist, bei der Anfälligkeit dafür mit einem ersten MHC-Typ-Allel assoziiert ist, und bei der die Nichtanfälligkeit dafür mit einem zweiten MHC-Typ-Allel assoziiert ist, das gegen die Erkrankung schützt, wobei das Verfahren darin besteht, zu bestimmen, ob ein Individuum das Anfälligkeit verleihende Allel aufweist, und dadurch gekennzeichnet ist, daß bei den genannten potentiell anfälligen Individuen bestimmt wird, ob ein Schutzallel abwesend ist.

7.  Verfahren nach Anspruch 6, worin die Autoimmunerkrankung IDDM ist, das Individuum ein Mensch ist und bestimmt wird, ob das Individuum ein Allel aufweist, das Anfälligkeit verleiht, wobei die Anfälligkeit verleihenden Allelen DR3 und DR4(3.2) umfassen, und ob dem Individuum ein Allel fehlt, das Schutz verleiht, wobei die Schutz verleihenden Allelen DR2 DQ1(Dw2) und DR2 DQ1(Dw12) umfassen.

8.  Verfahren zur Herstellung eines Polypeptids, das ein Schutzepitop gegen eine Autoimmunerkrankung bei einem Individuum enthält, für die das Individuum anfällig ist, wobei die Autoimmunerkrankung eine ist, bei der Anfälligkeit dafür mit einem ersten MHC-Allel assoziiert ist, gekennzeichnet durch das Identifizieren eines zweiten MHC-Typ-Allels, das gegen die Erkrankung schützt, und das Synthetisieren des Polypeptids durch chemische Mittel, wobei die Aminosäuresequenz für das von der DNA-Sequenz des Schutzallels abgeleitete Epitop verwendet wird.

9.  Verfahren zur Herstellung eines Polypeptids, das ein Schutzepitop gegen eine Autoimmunerkrankung bei einem Individuum enthält, für die das Individuum anfällig ist, wobei die Autoimmunerkrankung eine ist, bei der die Anfälligkeit dafür mit einem ersten MHC-Allel assoziiert ist, gekennzeichnet durch das Identifizieren eines zweiten MHC-Typ-Allels, das gegen die Erkrankung schützt, und das Exprimieren eines für das genannte Polypeptid kodierenden rekombinanten Vektors in einem kompatiblen Wirt und das Isolieren des genannten Polypeptids.

10. Verfahren nach Anspruch 8 oder 9, worin das Individuum ein Mensch ist, die Autoimmunerkrankung IDDM ist und worin für die Aminosäuresequenz des Schutzepitops innerhalb von DR2 DQ1 (Dw2) oder DR2 DQ1 (Dw12) oder DR4 DQw 3.1 kodiert wird.

**Revendications**

1.  Composition pharmaceutique pour traiter de façon prophylactique un individu contre une maladie auto-immune à laquelle l'individu est sensible, la maladie auto-immune étant une maladie à laquelle une sensibilité est associée à un premier type d'allèle MHC, et à laquelle une non sensibilité est associée à un second type d'allèle MHC qui est protecteur contre la maladie, la composition pharmaceutique comprenant une quantité rendant tolérant d'un antigène encodé dans l'allèle protecteur, dans laquelle l'antigène provoque l'effet protecteur.

2.  Composition pharmaceutique pour traiter de façon prophylactique un humain contre l'IDDM à laquelle l'humain est sensible, la sensibilité étant associée à un premier type d'allèle HLA, et dans laquelle un second allèle HLA est protecteur contre l'IDDM, la composition pharmaceutique comprenant une quantité rendant tolérant d'un antigène encodé dans l'allèle protecteur HLA, dans laquelle l'antigène provoque l'effet protecteur.

3. Composition selon la revendication 2, dans laquelle l'antigène protecteur est encodé dans DR2 DQ1 (Dw2) ou DRZ DQ1 (Dw12) ou DR4 DQw 3.1.

4. Composition selon la revendication 2, dans laquelle l'antigène est un fragment protecteur ou un mutant d'un antigène protecteur encodé dans l'allèle protecteur HLA, dans laquelle ledit fragment protecteur ou ledit mutant contient une séquence amino-acide qui forme un déterminant antigénique qui est fortement réactif par réaction croisée avec ce déterminant antigénique dans la séquence protectrice HLA qui provoque l'effet protecteur.

5. Composition selon la revendication 2, 3, 4, dans laquelle l'antigène est un polypeptide composé de la séquence amino acide

$$N\text{-pro-gln-gly-arg-pro-asp-ala-glu-tyr-C}$$
$$ou$$
$$N\text{-pro-leu-gly-pro-pro-asp-ala-glu-tyr-C}$$
$$ou$$
$$N\text{-pro-gln-gly-arg-ser-asp-ala-glu-tyr-C}$$

dans laquelle ledit polypeptide contient une séquence amino acide qui forme un déterminant antigénique qui est fortement réactif par réaction croisée avec ce déterminant antigénique dans la séquence protectrice HLA qui provoque l'effet protecteur.

6. Procédé pour déterminer si un individu doit être traité de façon prophylactique contre une maladie auto-immune, la maladie autoimmune étant l'une de celles à laquelle une sensibilité est associée à un premier type d'allèle MHC, et à laquelle une non susceptibilité est associée avec un second type d'allèle MHC qui est protecteur contre la maladie, le procédé consiste à déterminer si un individu possède l'allèle conférant la sensibilité, et étant caractérisé en ce que, pour ces dits individus potentiellement sensibles, à déterminer si un allèle protecteur est absent.

7. Procédé selon la revendication 6, dans lequel la maladie autoimmune est l'IDDM, l'individu est un humain, et on détermine si l'individu a l'allèle conférant la sensibilité, les allèles conférant la sensibilité comprenant le DR3 et le DR4 (3.2), et si l'individu manque d'un allèle conférant la protection, les allèles conférant la protection comprenant le DR2 DQ1 (Dw2) et le DR2 DQ1 (Dw12).

8. Procédé pour préparer un polypeptide comprenant un déterminant antigénique protecteur contre une maladie auto-immune chez un individu à laquelle l'individu est sensible, la maladie auto-immune étant l'une de celles à laquelle une sensibilité est associée à un premier type d'allèle MHC, caractérisé en ce qu'on identifie un second type d'allèle MHC qui est protecteur contre la maladie, et qu'on synthétise le polypeptide par des moyens chimiques, en utilisant la séquence amino acide pour le déterminant antigénique dérivé de la séquence ADN de l'allèle protecteur.

9. Procédé pour préparer un polypeptide contenant un déterminant antigénique protecteur contre une maladie auto-immune chez un individu à laquelle l'individu est sensible, la maladie auto-immune étant l'une de celles à laquelle une sensibilité est associée à un premier type d'allèle MHC, caractérisé en ce qu'on identifie un second type d'allèle MHC qui est protecteur contre la maladie, et en ce qu'on exprime un vecteur recombinant encodant ledit polypeptide dans un hôte compatible, et en ce qu'on isole ledit polypeptide.

10. Procédé selon la revendication 8 ou 9, dans lequel l'individu est un humain, la maladie auto-immune est l'IDDM, et dans lequel la séquence amino acide du déterminant antigénique protecteur est encodé dans DR2 DQ1 (Dw2) ou DR2 DQ1 (Dw12) ou DR4 DQw 3.1.

EP 0 286 447 B1

# FIG. I

DR4 DQß Alleles

First Domains DQβ3.1 and DQβ3.2

DQß3.2 RDSPEDFVYQFKGMCYFTNGTERVRLVTRYIYNREEYARFDSDVGVYRAAVTPLGPPAAEYWNSQKEVLERTRAELDTVCRHNYQLELRTTLQRR

DQß3.1 RDSPEDFVYQFKAMCYFTNGTERVRYVTRYIYNREEYARFDSDVEVYRAAVTPLGPPDAEYWNSQKEVLERTRAELDTVCRHNYQLELRTTLQRR

# FIGURE 2A

DQβ: Protein Sequences of First Domains

```
                        10                20                30                40                50                60
DR1 DQw1.1     R D S P E D F V Y Q  F K G L C Y F T N G  T E R V R G V T R H  I Y N R E E Y V R F  D S D V G V Y R A V  T P Q G R P V A E Y
DR2 Dw2 DQw1.2 · · · · · · · F ·    · · · M · · · · · ·  · · · · L · · · Y    · · · · · · A · ·    · · · · · · · · · ·  · · · · · · D · · ·
DR2 Dw12       · P · · · · · L ·    · · A M · · · · · ·  · · · · Y · · · Y    · · · · · · D · · ·  · · · · · · · · · ·  · · · · · · D · · ·
DR2 AZH        · · · · · · · · ·    · · · · · · · · · ·  · · · · · · · · ·    · · · · · · · · · ·  · · · · · · · · · ·  · · · · · · S · · ·
DR3 DQw2       · · · · · · · · ·    · · · M · · · · · ·  · · · · L · S · S    · · · · · · · I · ·  · · · · E F · · ·    · L L · L · A · · ·
DR4 DQw3.1     · · · · · · · · ·    · · A M · · · · · ·  · · · · Y · · · Y    · · · · · · A · ·    · · · · E · · · · ·  · · L · P · D · · ·
DR4 DQw3.2     · · · · · · · · ·    · · · M · · · · · ·  · · · · L · · · Y    · · · · · · A · ·    · · · · · · · · · ·  · · L · P · A · · ·
DR4 DQBLANK    · · · · · · · F ·    · · · M · · · · · ·  · · L · · · · · Y    · · · · · · A · ·    · · · · · · · · · ·  · · L · · L D · · ·
DR5 DQw3.1     · · · · · · · · ·    · · A M · · · · · ·  · · · · Y · · · Y    · · · · · · A · ·    · · · · E · · · · ·  · · L · P · D · · ·
DR6 DQw1.6     · · · · · · · · ·    · · · · · · · · · ·  · · · · · · · · ·    · · · · · · · · · ·  · · · · · · · · · ·  · · · · · · · · · ·
DR7 DQw2       · · · · · · · · ·    · · · M · · · · · ·  · · · · L · S · S    · · · · · · · I · ·  · · · · E F · · ·    · L L · L · A · · ·
DR8 DQ BLANK   · · · · · · · F ·    · · · M · · · · · ·  · · L · · · · · Y    · · · · · · A · ·    · · · · · · · · · ·  · · L · · L D · · ·
DR9 DWw3.3     · · · · · · · · ·    · · · M · · · · · ·  · · · · L · · · Y    · · · · · · · · · ·  · · · · · · · · · ·  · · L · P · D · · ·

                        70                80                90
DR1 DQw1.1     W N S Q K E V L E G  A R A S V D R V C R  H N Y E V A Y R G I  L Q R R
DR2 Dw2 DQw1.2 · · · · · · · · · ·  T · · E L · T · · ·  · · · · · · F · · ·  · · · ·
DR2 Dw12       · · · · · · L · · R  T · · E L · T · · ·  · · · · · · F · · ·  · · · ·
DR2 AZH        · · · · · · · · · ·  · · · · · · · · · ·  · · · · · · · · · ·  · · · ·
DR3 DQw2       · · · · · · L · · R  K · · A · · · · · ·  · · · Q L E L · T T  · · · ·
DR4 DQw3.1     · · · · · · · · · R  T · · E L · T · · ·  · · · Q L E L · T T  · · · ·
DR4 DQw3.2     · · · · · · · · · R  T · · E L · T · · ·  · · · Q L E L · T T  · · · ·
DR4 DQBLANK    · · · · · · L · · E  D · · · · · T · · ·  · · · Q L E L · T T  · · · ·
DR5 DQw3.1     · · · · · · · · · R  T · · E L · T · · ·  · · · Q L E L · T T  · · · ·
DR6 DQw1.6     · · · · · · · · · ·  · · · · · · · · · ·  · · · · · · · · · ·  · · · ·
DR7 DQw2       · · · · · · L · · R  K · · A · · · · · ·  · · · Q L E L · T T  · · · ·
DR8 DQ BLANK   · · · · · · L · · E  D · · · · · T · · ·  · · · Q L E L · T T  · · · ·
DR9 DWw3.3     · · · · · · · · · R  T · · E L · T · · ·  · · · Q L E L · T T  · · · ·
```

The dashes indicate amino acids which are the same as in DR1 DQw1.1

Figure 2B

| Type | HLA-Type | Sequence |
|---|---|---|
| Suscept | DR4-3.2 | 36 Glu Tyr Ala Arg 40 Phe Asp Ser Asp Val 45 Gly Val Tyr Arg Ala 50 Val Thr Pro<br>57 Leu Gly Pro Pro Ala 60 Ala Glu Tyr Trp Asn Ser Gln Lys Glu Val Leu |
| | DR3-DQW2 | 36 Glu Ile Val Arg 40 Phe Asp Ser Asp Val 45 Gly Glu Phe Arg Ala 50 Val Thr Leu<br>57 Leu Gly Leu Pro Ala 60 Ala Glu Tyr Trp Asn Ser Gln Lys Asp Ile Leu |
| Non-Suscept | DR4-3.1 | 36 Glu Tyr Ala Arg 40 Phe Asp Ser Asp Val 45 Gly Val Tyr Arg Ala 50 Val Thr Pro<br>57 Leu Gly Pro Pro Asp 60 Ala Glu Tyr Trp Asn Ser Gln Lys Glu Val Leu |
| Non-Prot | DR2-DQ1 (AZH) | 36 Glu Tyr Val Arg 40 Phe Asp Ser Asp Val 45 Gly Val Tyr Arg Ala 50 Val Thr Pro<br>57 Gln Gly Arg Pro Ser 60 Ala Glu Tyr Trp Asn Ser Gln Lys Glu Val Leu |
| | DR1-DQ1 | 36 Glu Tyr Val Arg 40 Phe Asp Ser Asp Val 45 Gly Val Tyr Arg Ala 50 Val Thr Pro<br>57 Gln Gly Arg Pro Val 60 Ala Glu Tyr Trp Asn Ser Gln Lys Glu Val Leu |
| Protected | DR2-DQ1 (Dw2) | 36 Glu Tyr Val Arg 40 Phe Asp Ser Asp Val 45 Gly Val Tyr Arg Ala 50 Val Thr Pro<br>57 Gln Gly Arg Pro Asp 60 Ala Glu Tyr Trp Asn Ser Gln Lys Glu Val Leu |
| Protected | DR2-DQ1 (Dw12) | 36 Glu Asp Ala Arg 40 Phe Asp Ser Asp Val 45 Gly Val Tyr Arg Ala 50 Val Thr Pro<br>57 Gln Gly Arg Pro Asp 60 Ala Glu Tyr Trp Asn Ser Gln Lys Glu Val Leu |

EP 0 286 447 B1

| | | | 36 | | | | 40 | | | | | 45 | | | | | 50 | | | |
|---|---|---|---|---|---|---|---|---|---|---|---|---|---|---|---|---|---|---|---|---|
| Mouse | NOD | | Glu | Tyr | Leu | Arg | Phe | Asp | Ser | Asp | Val | Gly | Glu | Tyr | Arg | Ala | Val | Thr | Glu |
| | | | | | | | 57 | | | 60 | | | | | | | | | |
| | | | Leu | Gly | Arg | His | Ser | Ala | Glu | Tyr | Tyr | Asn | Lys | Gln | * | Tyr | * | Leu | |

| | | | 36 | | | | 40 | | | | | 45 | | | | | 50 | | | |
|---|---|---|---|---|---|---|---|---|---|---|---|---|---|---|---|---|---|---|---|---|
| | f | | Glu | Tyr | Leu | Arg | Phe | Asp | Ser | Asp | Val | Gly | Glu | Tyr | Arg | Ala | Val | Thr | Glu |
| | | | | | | | 57 | | | 60 | | | | | | | | | |
| | | | Leu | Gly | Arg | Ser | Asp | Ala | Glu | Tyr | Tyr | Asn | Lys | Gln | * | Tyr | * | Leu | |

| | | | 36 | | | | 40 | | | | | 45 | | | | | 50 | | | |
|---|---|---|---|---|---|---|---|---|---|---|---|---|---|---|---|---|---|---|---|---|
| | u | | Glu | Tyr | Leu | Arg | Phe | Asp | Ser | Asp | Val | Gly | Glu | Tyr | Arg | Ala | Val | Thr | Glu |
| | | | | | | | 57 | | | 60 | | | | | | | | | |
| | | | Leu | Gly | Arg | Pro | Asp | Ala | Glu | Tyr | Tyr | Asn | Lys | Gln | * | Tyr | * | Leu | |

| | | | 36 | | | | 40 | | | | | 45 | | | | | 50 | | | |
|---|---|---|---|---|---|---|---|---|---|---|---|---|---|---|---|---|---|---|---|---|
| | d | | Glu | Tyr | Val | Arg | Phe | Asp | Ser | Asp | Val | Gly | Glu | Tyr | Arg | Ala | Val | Thr | Glu |
| | | | | | | | 57 | | | 60 | | | | | | | | | |
| | | | Leu | Gly | Arg | Pro | Asp | Ala | Glu | Tyr | Trp | Asn | Ser | Gln | Pro | Glu | Ile | Leu | |